# EUROPEAN PATENT SPECIFICATION

(11) **EP 2 990 028 B1**
(45) Date of publication and mention of the grant of the patent: **13.06.2018**
(21) Application number: 14787728.6
(22) Date of filing: 22.04.2014
(51) Int. Cl.: A61K 8/73, A61K 8/02, A61K 8/06, A61K 8/19, A61K 47/04, A61K 47/38, A61Q 19/00, C09K 3/00, A61K 8/24, A61K 8/27, A61K 8/365, A61K 8/44, A61K 8/46, A61K 8/60, A61K 8/67, A61K 8/81, A61K 8/20, A61K 8/23, A61K 45/06, A61L 26/00, A61K 31/717

(54) **THICKENING COMPOSITION**
VERDICKUNGSZUSAMMENSETZUNG
COMPOSITION ÉPAISSISSANTE

(30) Priority: 22.04.2013 JP 2013089702
(43) Date of publication of application: 02.03.2016
(73) Proprietor: Nissan Chemical Industries, Ltd., Chiyoda-ku Tokyo 101-0054 (JP)
(72) Inventor: HAYASHI, Hisato, Funabashi-shi Chiba 274-8507 (JP); IWAMA, Takehisa, Funabashi-shi Chiba 274-8507 (JP)
(74) Representative: Hoffmann Eitle
(86) International application number: PCT/JP2014/061338
(87) International publication number: WO 2014/175289

(56) References cited:
- EP-A1- 1 839 499
- EP-A1- 2 929 874
- WO-A1-2006/062089
- WO-A1-2014/088034
- JP-A- S57 107 234
- JP-A- 2008 106 178
- JP-A- 2012 193 139
- US-A- 4 378 381

## Description

### TECHNICAL FIELD

The present invention relates to a thickening composition, and in particular, to a thixotropic thickening composition that, when added, does not exhibit a significant increase in thickening, and that exhibits gel-forming ability with a variety of electrolyte additives while maintaining low viscosity, gel-forming in any pH of a solution, sprayability, excellent emulsion stability, as well as to cosmetics, external preparations, skin protective agents, and wound dressings containing the same.

### BACKGROUND ART

Cosmetics can be in a wide variety of forms such as liquid, emulsion, gel, cream, and stick. Various thickening agents and gelators have been used for improvement of feel of use and retention of the features of products. Conventional aqueous thickening gelators for cosmetics include water-soluble polymers, for example, natural polymers such as sodium hyaluronates, sodium alginates, and xanthan gums; semisynthetic polymers such as hydroxyethyl celluloses and carboxymethyl celluloses; synthetic polymers such as carboxyvinyl polymers, polyvinyl alcohols, and sodium polyacrylates, and are appropriately selected and used based on the purpose and effect desired.

However, there have been such problems in that the viscosity of cosmetics rapidly decreases due to electrolytes such as sweat when applied to skin and the cosmetics fall off from skin and that the cosmetics are difficult to apply due to electrolytes such as sweat, because thickening gelators for cosmetics, for example, carboxyvinyl polymers that are mainly used, are often ionic. In an effort to solve these problems, attempts have been made to increase salt resistance and provide excellent feel of use, and for example, aqueous gel cosmetics containing a carboxyvinyl polymer, a basic substance, a hydrophobic anhydrous silicic acid, and a polyalcohol (refer to e.g., Patent Document 1) have been proposed.

In addition, gel cosmetics containing low crystalline regenerated cellulose obtained by sulfuric acid treatment, a vegetable-based oil, and a nonionic surfactant (refer to Patent Document 2) have been proposed. Patent Document 2 states that in order to obtain thixotropy enough to spray the gel cosmetics in the form of mist, preferred are cellulose particulates with an average polymerization degree of 100 or less, a fraction of 0.1 or less of a cellulose I type crystalline component, and a fraction of 0.4 or less of a cellulose II type crystalline component.

Furthermore, attempts have been made to increase a moisture retaining property and reduce stickiness with cosmetics containing cellulose fibers obtained without chemical treatment of vegetable-based cellulose and a polyalcohol (refer to Patent Document 3).

In addition, syntheses of novel polysaccharide derivatives have been reported, in which hydroxy groups of polysaccharides are substituted by groups of a second family or a third family in a predetermined ratio (refer to Patent Document 4). Attempts have been made to increase viscosity at low concentration by introducing hydroxy groups, fluoro alkyl groups, and sulfone groups as substituents into them and causing the intramolecular and intermolecular self-assembly of the polysaccharide derivative, as well as imparting salt resistance with strong acid groups.

Furthermore, xanthan gums, which are microorganism polymers, are less temperature dependent and more stable in a wide range of pHs than other thickening agents, and exhibit salt resistant, but sometimes significantly impair feel of use for products as a result of sliminess and stickiness due to part of their properties. In order to improve impaired feel of use, attempts have been made to chemically modify functional groups (refer to Patent Document 5).

US 4.378.381 A describes an edible suspension of a finely divided food material in a liquid suspending medium which swells cellulose, the suspension also containing microfibrillated cellulose in an amount sufficient to produce a stable, homogeneous suspension.

EP 1 839 499 A1 describes a composition comprising a polysaccharide and a highly dispersible cellulose complex composed of a hydrophilic substance, a water-soluble polymer and a water-dispersible cellulose being fine-fibrous cellulose from plant cell walls as a raw material, as well as a stabilizer and thickening agent comprising the composition.

EP 2 929 874 A1 describes a cosmetic additive containing cellulose fibers having an average fiber diameter (D) of 0.001 to 0.05 µm, and a ratio (L/D) of average fiber length (L) to average fiber diameter (D) of 5 to 500, and a cosmetic containing the cosmetic additive.

JP 2008 106178 A describes a dried composition comprising 1-49 mass% crystalline fine fibrous cellulose made from plant cell wall and 51-99 mass% water-soluble polymer, wherein, as the water-soluble polymer, carboxymethylcellulose sodium and dextrin, or xanthane gum and dextrin are preferably used. Also described is the addition of a polysaccharide thereto to obtain a thickening or gelling agent.

### Prior Art Documents

### Patent Documents

Patent Document 1: Japanese Patent No. 2913514 (JP 2913514 B1)
Patent Document 2: Japanese Patent No. 5002433 (JP 5002433 B1)
Patent Document 3: Japanese Patent Application Publication No. 2012-193139 (JP 2012-193139A)
Patent Document 4: Japanese Patent No. 3954110 (JP 3954110 B1)
Patent Document 5: Japanese Patent Application Publication No. H11-302303 (JP 11-302303 A)
Patent Document 6: Japanese Patent Application Publication No. 2000-72643 (JP 2000-72643 A)

### SUMMARY OF THE INVENTION

### Problem to be Solved by the Invention

As described above, previously various proposals have been made to improve the performance, feel of use, and the like of thickening gelators for cosmetics. However, for example, aqueous gel cosmetics proposed in Patent Document 1 fail to satisfy desired properties required for cosmetics in, for example, stickiness and coating properties when applied. In addition, for gel cosmetics containing low crystalline regenerated cellulose obtained by sulfuric acid treatment, which are described in Patent Document 2, there are such problems in that it involves laborious steps requiring a special mixer when blended with other components or when diluted, and exhibits low heat resistance, and low salt resistance, for example, decreased dispersibility when blended with an ionic component, resulting in the occurrence of white turbidity or precipitation, or separation of the cosmetics.

For cosmetics containing cellulose fibers obtained without chemical treatment, which are described in Patent Document 3, the problems lie in complication of formulation and feel of use, for example, the cellulose fibers have high aspect ratios (L/D) and extremely long fiber lengths from several tens to several thousands of µm, which reduces transparency when added to cosmetics, and also causes dry skin and clumping when applied alone. Therefore, the cellulose fibers should be blended with other components to prevent these.

For polysaccharide derivatives into which hydroxy groups, fluoro alkyl groups, and sulfone groups are introduced, which are described in Patent Document 4, they exhibit excellent thickening properties and high salt resistance, but there are such problems in that when applied to cosmetic products and quasi-drugs, etc., blend of a novel compound is withheld and skin irritancy may occur due to the introduction of strong acid groups.

For acetylated xanthan gums obtained by chemically modifying xanthan gums that are salt resistant and highly stable for pH, which are described in Patent Document 5, they exhibit improved feel of use (see Patent Document 6), but there are such problems in that concentrated sulfuric acid or acetic anhydride is used for production and it is difficult to highly purify these by industrial processes, and thus these materials remaining in products can cause skin irritancy.

It is an object of the present invention to provide a thickening composition that exhibits an effect of thickening and gelating to solve these problems, in particular, a thixotropic thickening composition that exhibits high salt resistance and heat resistance, and that can be easily blended with cosmetics without laborious steps, and that also exhibits gel-forming ability with a variety of electrolyte additives while maintaining low viscosity and gel-forming ability in any pH of a solution, and that exhibit sprayability and excellent emulsion stability, as properties conventionally required for thickening compositions, as well as cosmetics, external preparations, skin protective agents, and wound dressings containing the same.

### Means for Solving the Problem

The inventors have made extensive investigations to solve the aforementioned problems and, as a result, found that highly dispersing cellulose fibers without dissolution of them in a medium, in a dispersion liquid containing an electrolyte component and cellulose fibers obtained by microprocesssing by high pressure grinding without chemical treatment such as surface modification and solubilization treatment and also in a dispersion liquid containing a polyalcohol provides thickening effect, excellent transparency as well as salt resistance and heat resistance, good feel of use on skin, reduced stickiness when dried, and excellent emulsion stability, and have thus completed the present invention.

Specifically, a first aspect of the present invention relates to a thickening composition comprising an electrolyte and cellulose fibers having an average fiber diameter (D) of 0.001 to 100 µm and containing hemicellulose and amorphous regions characterized in that a ratio (L/D) of an average fiber length (L) to the average fiber diameter (D) is 5 to 500, and in that the cellulose is derived from broadleaf trees.

A second aspect relates to the thickening composition according to the first aspect, in which the electrolyte is one or two or more selected from the group consisting of sodium chloride, potassium chloride, zinc chloride, calcium chloride, magnesium chloride, sodium dihydrogenphosphate, sodium hydrogencarbonate, sodium sulfite, sodium sulfate, glucosamine hydrochloride, dipotassium glycyrrhizinate, disodium dihydrogen ethylenediamine tetraacetate dihydrate, sodium citrate dihydrate, sodium ascorbate, magnesium L-ascorbyl 2-phosphate, sodium lactate, sodium dodecylbenzenesulfonate, sodium lauryl sulfate, lauramidopropyl betaine, lauryldimethylaminoacetic acid betaine, lauryl hydroxy sulfobetaine, lauryl sodium aspartate, hypromellose, carbomers, polyacrylic acids, chitosans, xanthan gums, propylene glycol alginate, carboxymethylcellulose, and salts thereof.

A third aspect relates to the thickening composition according to the first aspect or the second aspect, comprising the cellulose fibers having an average fiber diameter (D) of 0.001 to 0.05 µm and a ratio (L/D) of an average fiber length (L) to the average fiber diameter (D) of 5 to 500.

A fourth aspect relates to the thickening composition according to the third aspect, characterized in that the cellulose fibers are cellulose processed by kraft pulping.

A fifth aspect relates to the thickening composition according to the fourth aspect, characterized in that the cellulose fibers are kraft pulp.

An sixth aspect relates to the thickening composition according to any one of the first aspect to the fifth aspect, comprising a proportion of 0.0001 part by mass to 30 parts by mass of the electrolyte with respect to 1 part by mass of the cellulose fibers.

A seventh aspect relates to the use of the thickening composition according to any one of the first aspect to the sixth aspect as an additive added to increase viscosity of a cosmetic.

A eight aspect relates to the use according to the seventh, characterized in that the thickening composition is an additive added to a cosmetic of a cosmetic product in which the cosmetic is sprayed in use.

An ninth aspect relates to the thickening composition according to any one of the first aspect to the sixth aspect, which exhibits thixotropy, and wherein the electrolyte is sodium lactate.

A tenth aspect relates to the thickening composition according to any one of the first aspect to the sixth and the ninth aspect, characterized in that in dynamic viscoelasticity measurements using a thickening composition containing 1% by mass of cellulose fibers and 0.01% by mass of an electrolyte, a dynamic viscosity coefficient η' measured in a cone plate at a dynamic displacement of 0.5 deg and a frequency of 1Hz in a static state at 25°C for 2 hours is at least 150% of η' at 25°C for 0 hour.

A eleventh aspect relates to a cosmetic comprising the thickening composition according to any one of the first aspect to the sixth aspect and the ninth aspect to the tenth aspect.

A twelfth aspect relates to the cosmetic according to the eleventh aspect, further comprising an aqueous component.

A thirteenth aspect relates to the cosmetic according to the eleventh aspect or the twelfth aspect, further comprising a proportion of 1 part by mass to 100 parts by mass of one or two or more polyalcohols selected from the group consisting of 1,3-butylene glycol, glycerol, and diglycerol with respect to 1 part by mass of the cellulose fibers.

A fourteenth aspect relates to the cosmetic according to any one of the eleventh aspect to the thirteenth aspect, further comprising an oil component.

A fifteenth aspect relates to the cosmetic according to any one of the eleventh aspect to the fourteenth aspect, further comprising a surfactant.

An sixteenth aspect relates to the cosmetic according to the fourteenth aspect or the fifteenth aspect, in which the cosmetic is in a form of emulsion.

A seventeenth aspect relates to an external preparation comprising the thickening composition according to any one of the first aspect to the sixth aspect and the ninth aspect to the tenth aspect.

### Effects of the Invention

The present invention provides a thickening composition that exhibits excellent transparency, feel of use on skin, and heat resistance, and that can be easily blended with cosmetics without laborious steps, and that also exhibits salt resistance and a thickening effect due to thixotropy, and that maintains a high thickening effect and excellent emulsion stability when blended with an electrolyte component, an oil component, or a polyalcohol, and that exhibits gel-forming ability in any pH of a solution and sprayability in the form of mist, as well as cosmetics and others containing the same.

### BRIEF DESCRIPTION OF THE DRAWINGS

[FIG. 1] FIG. 1 is a chart showing results of viscosity measurements with a tuning fork vibration for test liquids in Example 2 and Comparative Examples 5 to 8.
[FIG. 2] FIG. 2 is a chart showing results of the evaluation on thixotropy for test liquids in Examples 31 and 32 and Comparative Examples 20 and 25.

### MODES FOR CARRYING OUT THE INVENTION

The notable characteristic of a thickening composition of the present invention is that the thickening composition contains cellulose fibers microprocessed by high pressure grinding without chemical treatment, exhibits a high thickening effect due to thixotropy in a wide range of pHs with the addition of an electrolyte component, and also exhibits an effect of an emulsion stabilizer that can highly disperse and stabilize components such as an oil component in a simple way.

In particular, a thickening composition of the present invention and cosmetics containing the same are characterized by exhibiting especially excellent transparency when added and good spreadability and feel of use when applied, because they contain relatively short cellulose fibers with an aspect ratio (L/D) of 5 to 500 compared with conventionally proposed cellulose fibers. In addition, they exhibit high salt resistance and a high thickening effect and excellent emulsion stability in a wide range of pHs, and reduce stickiness when dried, and can be sprayed in the form of mist in use. Hereinafter, the present invention will be described in detail.

### <Thickening compositions>

### [Cellulose materials]

Cellulose fiber materials used in a thickening composition of the present invention include cellulose derived from derived from broadleaf trees.

Cellulose derived from broadleaf trees includes a higher order structure having fibril, lamella, and desmacyte in a stepwise manner with bundles of very thin fibers called microfibrils, as opposed to bacteria cellulose, which includes a fine network structure with microfibrils of cellulose secreted by bacteriocyte forms by the same thickness.

A crystalline portion of the above broadleaf tree-derived cellulose has the cellulose I type crystal form, and the crystallinity largely varies depending on cellulose sources. Broadleaf tree-derived cellulose includes a higher order structure containing hemicellulose, lignin, and the like as an impurity. Thus, the crystallinity of purified pulp made from these materials is less than 80%. In contrast, a high crystallinity of 80% or more would be obtained for purified pulp made from a material from among Cladophoraceae, bacteria cellulose, Glaucocystis, or ascidian cellulose. Cellulose derived from broadleaf trees is used in view of the storage stability of an aqueous dispersion liquid.

Preferably, cellulose fiber materials used in a thickening composition of the present invention include cellulose with a crystallinity of less than 80%.

### [Methods for grinding cellulose]

In the present invention, cellulose fibers obtained by grinding the above cellulose materials are used. Methods for grinding cellulose materials preferably include, but are not particularly limited to, methods for utilizing a high shearing force such as a high pressure homogenizer, a grinder (a stone mill), or a mill for stirring media such as a bead mill in an attempt to micronize them to obtain fiber diameters and fiber lengths as described below for the purposes of the present invention.

Among them, preferably a high pressure homogenizer is used to micronize them, and preferably for example, a wet grinding method as disclosed in Japanese Patent Application Publication No.2005-270891 (JP 2005-270891 A) is used to micronize (grind) them. Specifically, a cellulose material is ground by injecting a dispersion liquid containing a dispersed cellulose material from a pair of nozzles at high pressure to effect collision, and for example, Starburst System (a high pressure grinding machine manufactured by Sugino Machine Limited) can be used to implement it.

When the above high pressure homogenizer is used to micronize (grind) cellulose materials, the degree of micronization and homogenization depends on a pressure for pumping to an ultra-high pressure chamber in the high pressure homogenizer, the number of times in which the cellulose materials are passed through the ultra-high pressure chamber (the number of processes), and the concentration of cellulose in an aqueous dispersion liquid.

Typically, a pumping pressure (a process pressure) ranges from 50 to 250 MPa, and preferably from 150 to 245 MPa. When a pumping pressure is less than 50 MPa, micronization of cellulose is not enough, whereby any effect desired from the micronization can not be obtained.

The concentration of cellulose in an aqueous dispersion liquid when micronized ranges from 0.1% by mass to 30% by mass, and preferably from 1% by mass to 10% by mass. If the concentration of cellulose in the aqueous dispersion liquid is less than 0.1% by mass, the productivity is significantly low, and if it is more than 30% by mass, the efficiency of grinding is too low to produce desired cellulose fibers.

The number of processes of micronizing (grinding) is not particularly limited but dependent on the concentration of cellulose in an aqueous dispersion liquid. For example, when the concentration of cellulose ranges from 0.1% by mass to 1% by mass, the micronization is performed well with the number of processes approximately from 10 to 100 times, when it ranges from 1% by mass to 10% by mass, from 10 to 1000 times, and preferably from 201 to 1000 times. In the case of a high concentration of more than 30%, the number of processes of more than several thousands is required, and the viscosity becomes high, causing problems when handling the cellulose materials, and as a result, it is unpractical in view of productivity.

The average fiber diameter (D) of cellulose fibers used in the present invention ranges from 0.001 to 100 µm, and preferably 0.001 to 0.05 µm, and more preferably 0.01 to 0.05 µm. If the average fiber diameter is less than 0.001 µm, the cellulose fibers are too fine to obtain the effects of the addition of the cellulose fibers, which does not lead to improvement of the properties of cosmetics containing them.

The aspect ratio (L/D) of cellulose fibers used in the present invention is determined from the average fiber length/the average fiber diameter, which ranges from 5 to 500, and preferably 10 to 500. If the aspect ratio is less than 5, dispersibility of fibers in a solution is not enough, resulting in insufficient coating when dried. If the aspect ratio is more than 500, this means the fiber length is extremely long, resulting in decrease in transparency, as well as degradation in transparency and feel of use when applied to skin and the occurrence of clumping.

In the present invention, the average fiber diameter (D) of cellulose was determined by the following: first, a collodion supporting film manufactured by Okenshoji Co., Ltd. was subjected to hydrophilization treatment with an ion cleaner (J I C-410) manufactured by JEOL Ltd. for 3 minutes, and a few drops of a cellulose dispersion liquid (diluted with ultrapure water) prepared in production examples was added thereto, and then the film was dried at room temperature. The film was observed with transmission electron microscope (TEM, H-8000) (10,000 magnification) manufactured by Hitachi, Ltd. with an acceleration voltage of 200 kV. A fiber diameter was measured individually for 200 to 250 of cellulose fibers (the number of samples) using the resulting images and the number mean value was defined as the average fiber diameter (D).

For the average fiber length (L), a cellulose dispersion liquid prepared in the production examples was diluted to 400 times volume with dimethyl sulfoxide (DMSO), and the cellulose was dispersed in the solution. The resultant mixture was casted on a silicon wafer pretreated with surface hydrophilization by concentrated sulfuric acid, and dried at 110°C for 1 hour to obtain a sample. A fiber length was measured individually for 150 to 250 of cellulose fibers (the number of samples) using images from the observation of the resulting samples with scanning electron microscope (SEM, JSM-7400F) (10,000 magnification) manufactured by JEOL Ltd., and the number mean value was defined as the average fiber length (L).

### [Electrolyte components]

Electrolyte components may be in the form of inorganic salt or organic salt, and also may be a low molecular weight compound or a high molecular weight compound. Components having water solubility and no skin irritancy are preferable. For example, components that can be added to external preparations, in particular, cosmetic products, external drugs, or quasi-drugs may be widely included.

Preferably the amount of an electrolyte component to be added ranges from 0.001% by mass to 30% by mass with respect to the total mass of the cosmetics.

In the present invention, preferably an electrolyte component is a substance that dissociates into a cation and an anion in an aqueous solution or a polar solvent.

Specifically, electrolyte components include inorganic salts or organic salts. Examples of preferred inorganic salts include inorganic salts such as sodium chloride, potassium chloride, calcium chloride, magnesium chloride, zinc chloride, aluminum chloride, calcium carbonate, sodium carbonate, potassium carbonate, magnesium carbonate, potassium sulfite, sodium sulfate, sodium hydrogensulfate, sodium sulfite, sodium hydrogensulfite, potassium sulfate, sodium sulfate, calcium sulfate, magnesium sulfate, zinc sulfate, aluminum sulfate, potassium phosphate, sodium phosphate, disodium hydrogenphosphate, and sodium dihydrogenphosphate.

Examples of preferred organic salts that are low molecular weight compounds include glycyrrhizic acid salts such as dipotassium glycyrrhizic acid salt; α-hydroxy acid salts such as aminocaproic acid, citric acid, salicylic acid, lactic acid, glycolic acid, and tartaric acid; amino acids and derivatives thereof such as serine, glycine, asparagine, aspartic acid, tranexamic acid, lysine, threonine, alanine, thyrosin, valine, leucine, proline, arginine, threonine, cysteine, cysteine, methionine, tryptophan, glutamic acid, and pyrrolidone carboxylic acid; vitamins such as ascorbate, sodium ascorbate, potassium ascorbate, magnesium ascorbate, sodium ascorbate ester, ascorbic acid phosphoric ester magnesium, ascorbic acid phosphoric ester calcium, sodium ascorbyl sulfate, magnesium ascorbyl sulfate, calcium ascorbyl sulfate, ascorbic acid glucoside (2-O-α-D-glucopyranosyl-L-ascorbic acid), ascorbic acid glucosamine, dehydroascorbic acid, vitamin B2, vitamin B6 ,vitamin B12, vitamin B13, biotin, pantothenic acid, niacin, folic acid, inositol, carnitine, thiamine, thiamine disulfide, fursultiamine, dicethiamine, bisbutythiamin, bisbentiamine, benfotiamine, thiamine monophosphate disulfide, cycotiamine, octotiamine, and prosultiamine; as well as disodium ethylenediaminetetraacetate, trisodium ethylenediaminetetraacetate, tetrasodium ethylenediaminetetraacetate, sodium benzoate, 2-hydroxy-4-methoxybenzophenone-5-sulfonate, adenosine-3'-5'-cyclic monophosphate, adenosine monophoshate, adenosine diphoshate, adenosine triphosphate and salts thereof; anionic surfactants such as fatty acid soaps (sodium laurate, sodium palmitate, sodium stearate, and the like), potassium lauryl sulfate, and alkyl sulfate triethanolamine ether; cationic surfactants such as stearyl chloride trimethylammonium, benzalkonium chloride, and lauryl amine oxide; ampholytic surfactants such as imidazoline-based ampholytic surfactants (2-cocoyl-2-imidazolinium hydroxide-1-carboxy ethyloxy disodium salt, and the like), betaine-based surfactants (alkyl betaine, amide betaine, sulfobetaine, and the like), and acyl methyl taurine.

Examples of preferred organic salts that are high molecular weight compounds include hyaluronic acid, gellan gum, deacylation gellan gum, rhamsan gum, diutan gum, xanthan gum, carrageenan, xanthan gum, hexuronic acid, fucoidan, pectin, pectic acid, pectinic acid, heparan sulfate, heparin, heparan sulfate, keratosulfate, chondroitin sulfate, dermatan sulfate, rhamnan sulfate, and salts thereof; alginate derivatives such as sodium alginate and propylene glycol alginate ester; methyl cellulose, ethyl cellulose, hydroxyethyl cellulose, hydroxypropylcellulose, carboxymethylcellulose and salts thereof such as sodium; cellulose derivatives such as methyl hydroxypropylcellulose, cellulose sodium sulfate, and dialkyl dimethylannmonium sulfate cellulose; chitosans, carboxyvinyl polymers, polyacrylic acids, and salts thereof such as sodium salts; an acrylic acid/(meth)acrylic acid ester copolymer, an acrylic acid/(meth)acrylic acid alkyl copolymer, cationized cellulose such as polyquaternium-10, a diallyl dimethylannmonium chloride/acrylamide copolymer such as polyquaternium-7, an acrylic acid/diallyl dimethylannmonium chloride copolymer such as polyquaternium-22, an acrylic acid/diallyl dimethylannmonium chloride/acrylamide copolymer such as polyquaternium-39, an acrylic acid/cationized (meth)acrylic acid ester copolymer, an acrylic acid/cationized (meth)acrylic acid amide copolymer, an acrylic acid/methyl acrylate/methacrylamidepropyl trimethylammonium chloride copolymer such as polyquaternium-47, a methacrylate chloride choline ester polymer, cationized dextran, cationized polysaccharides such as guar hydroxypropyl trimonium chloride, polyethyleneimine, and a copolymer of a 2-methacryloyloxyethyl phosphorylcholine polymer and a butyl (meth)acrylate copolymer such as polyquaternium-51.

Among them, more preferred is one or two or more selected from the group consisting of sodium chloride, potassium chloride, zinc chloride, calcium chloride, magnesium chloride, sodium dihydrogenphosphate, sodium hydrogencarbonate, sodium sulfite, sodium sulfate, glucosamine hydrochloride, dipotassium glycyrrhizate, disodium dihydrogen ethylenediamine tetraacetate dihydrate, sodium citrate dihydrate, sodium ascorbate, magnesium L-ascorbyl-2-phosphate, sodium lactate, sodium dodecylbenzenesulfonate, sodium lauryl sulfate, lauramidopropyl betaine, lauryldimethylaminoacetic acid betaine, lauryl hydroxy sulfobetaine, lauryl sodium aspartate, hypromellose, carbomers, polyacrylic acids, chitosans, xanthan gums, propylene glycol alginate, carboxymethylcellulose, and salts thereof.

In the present invention, water solubility means that a component can dissolve in purified water at 25°C in at least an amount of 0.1% by mass.

Polar solvents may include alcohols such as methanol, ethanol, 1-propanol, 2-propanol, and 1-butanol, tetrahydrofuran, acetone, acetonitrile, dimethyl sulfoxide, and N, N- dimethylformamide, and these solvents can be used alone or as mixtures with water or as mixtures with other polar solvents. In view of the addition to cosmetics and the like, as polar solvents, preferred are alcohols, and more preferably methanol, ethanol, and 2-propanol.

### [Polyalcohols]

Polyalcohols that can used in the present invention include, but are not particularly limited to, for example, ethylene glycol, diethylene glycol, triethylene glycol, polyethylene glycol (weight average molecular weight: 1,500 or less), trimethylene glycol, propylene glycol, dipropylene glycol, polypropylene glycol (weight average molecular weight: 1,500 or less), glycerol, diglycerol, triglycerol, polyglycerin >3 in polymerization degree, 1,3-butanediol, 3-methyl-1,3-butanediol, 1,3-butylene glycol, 1,3-propanediol, 1,2-pentanediol, 1,5-pentanediol, 1,6-hexanediol, 1,7-heptanediol, 1,8-octanediol, 1,9-nonanediol, 1,10-decanediol, 1,11-undecanediol, 1,12-dodecanediol, 1,13-tridecanediol, 1,14-tetradecanediol, 1,15-pentadecanediol, 1,16-hexadecanediol, 1,17-heptadecanediol, 1,18-octadecanediol, 1,19-nonadecanediol, 1,20-icosanediol, 1,2-octanediol, 1,2-decanediol, 1,2-dodecanediol, 1,2-tetradecanediol, 1,2-hexadecanediol, 1,2-octadecanediol, and trimethylolpropane. In the present invention, preferably they are used in combination with one or more of these.

Among them, preferably 1,3-butylene glycol, glycerol, or diglycerol is used.

Desirably, polyalcohols are used in an amount to be added of 1 part by mass to 100 parts by mass, and preferably 1 part by mass to 20 parts by mass with respect to 1 part by mass of the cellulose fibers.

The above-described thickening composition of the present invention is not particularly limited, but may preferably exhibit thixotropy.

The thickening composition of the present invention is not particularly limited, but for example, when dynamic viscoelasticity is measured using a thickening composition containing 1% by mass of cellulose fibers and 0.01% by mass of an electrolyte, a dynamic viscosity coefficient η' measured in a cone plate at a dynamic displacement of 0.5 deg and a frequency of 1Hz in a static state at 25°C for 2 hours is at least 150%, and preferably at least 200%, and more preferably at least 300% of η' at 25°C for 0 hour.

The above-mentioned thickening composition of the present invention is suitable as an additive added to increase the viscosity of cosmetics, i.e., a thickener.

The thickening composition of the present invention is suitable as an additive that is added, in particular to a cosmetic of a cosmetic product in which the cosmetic is sprayed in use, i.e., as an additive for spray cosmetics, and the addition of the composition provides excellent spreading properties leading to cosmetic sprays with excellent spray performance to improve feel of use and spreadability.

### <Cosmetics>

The present invention also relates to cosmetics containing the above-mentioned thickening composition. The cosmetics can contain an aqueous component, an oil component, a surfactant, and the like.

A cosmetic of the present invention can be in a wide variety of forms such as toner (lotion), emulsion, cream, gel, and the like.

External preparations containing the above-mentioned thickening composition are also within the scope of the present invention, and can contain the same components as the cosmetics.

### [Aqueous components]

Water, alcohol, and a solvent mixture of water and alcohol can be used as the above-mentioned aqueous component. Preferred is water or a solvent mixture of water and alcohol, and more preferred is water.

Examples of the water are not particularly limited as long as it is used for cosmetic products, and may include deionized water, purified water, refined water, natural water, hot spring water, deep sea water, and steam distilled water (fragrant distilled water) obtained by steam distillation of plants and the like.

The alcohol is preferably water-soluble alcohol freely soluble in water, and more preferably alcohol having 1 to 6 carbon atoms, and even more preferably, ethanol, 2-propanol, or i-butanol, and most preferably ethanol or 2-propanol.

### [Oil components]

Oil components that can used in a cosmetic of the present invention are not particularly limited, and may include oils and fats derived from plants such as olive oil, jojoba oil, castor oil, soybean oil, rice oil, rice embryo oil, coconut oil, palm oil, cacao oil, meadowfoam oil, shear butter, tea tree oil, avocado oil, macademia nut oil, and olive squalane; oils and fats derived from animals such as squalane, mink oil, and turtle oil; waxes such as beeswax, carnauba wax, rice wax, and lanolin; hydrocarbons such as liquid paraffin, vaseline, and paraffin wax; fatty acids such as myristic acid, palmitic acid, stearic acid, oleic acid, isostearic acid, and cis-11-eicosenic acid; higher alcohols such as lauryl alcohol, cetanol, and stearyl alcohol; synthetic esters and synthetic triglycerides such as isopropyl myristate, isopropyl palmitate, butyl oleate, 2-ethyl hexyl glyceride, and higher fatty acid octyl dodecyl (stearic acid octyl dodecyl and the like).

Preferably the amount of oil components to be added ranges from 0.01% by mass to 99% by mass with respect to the total mass of the cosmetic.

### [Surfactants]

Surfactants that can be used in a cosmetic of the present invention include, but are not particularly limited to, for example, sorbitan fatty acid esters such as sorbitan monoisostearate, sorbitan monolaurate, sorbitan monopalmitate, sorbitan monostearate, penta-2-ethyl hexyl acid diglycerol sorbitan, and tetra-2-ethyl hexyl acid diglycerol sorbitan; glyceryl fatty acid esters such as monostearin acid glyceryl and glyceryl monostearate malic acid; polyglyceryl fatty acid esters such as monostearic acid polyglyceryl, monoisostearic acid polyglyceryl, diisostearic acid polyglyceryl, monlauric acid polyglyceryl, monooleic acid polyglyceryl, and monomyristic acid polyglyceryl; polyglyceryl fatty acids such as monostearic acid polyglyceryl, monoisostearic acid polyglyceryl, diisostearic acid polyglyceryl, monlauric acid polyglyceryl, monooleic acid polyglyceryl, and glyceryl fatty acid esters; propylene glycol fatty acid esters such as monostearin acid propylene glycol; hydrogenated castor oil derivatives such as polyoxyethylene hydrogenated castor oils such as polyoxyethylene hydrogenated castor oil 40 (HCO-40), polyoxyethylene hydrogenated castor oil 50 (HCO-50), polyoxyethylene hydrogenated castor oil 60 (HCO-60), and polyoxyethylene hydrogenated castor oil 80; polyoxyethylene sorbitan fatty acid esters such as monolauryl acid polyoxyethylene (20) sorbitan (polysorbate 20), monostearic acid polyoxyethylene (20) sorbitan (polysorbate 60), monooleic acid polyoxyethylene (20) sorbitan (polysorbate 80), and isostearic acid polyoxyethylene (20) sorbitan; polyoxyethylene monococonut oil fatty acid glyceryl; glycerol alkylethers; alkyl glucosides; polyoxyalkylene alkylethers such as polyoxyethylene lauryl ether, polyoxyethylene cetyl ether, polyoxyethylene stearyl ether, polyoxyethylene oleyl ether, and polyoxyethylene behenyl ether; and silicone surfactants such as polyoxyethylene/methyl polysiloxane copolymer, lauryl PEG-9 polydimethyl siloxy ethyl dimethicone, and PEG-9 polydimethyl siloxy ethyl dimethicone.

Preferably the amount of surfactants to be added ranges from 0.001% by mass to 20% by mass with respect to the total mass of the cosmetic.

### [Other components]

A thickening composition of the present invention and cosmetics containing the same can contain a functional additive as other component materials together with cellulose fibers, an electrolyte component, a polyalcohol, an oil component, and a surfactant. The functional additives include, for example, astringents, disinfection/antimicrobial agents, whitening agents, ultraviolet absorbers, moisturizers, cell activators, antiphlogistic/anti-allergic agents, antioxidant/active oxygen-removing agents, oils and fats, waxes, hydrocarbons, fatty acids, esters, perfumes, organic fine particles, inorganic fine particles, deodorants, and organic solvents.

These can be used alone or in combination of two or more of them.

### Examples

Hereinafter, the features of the present invention will be described as follows in more detail by referring to examples and comparative examples. The material, use amount, percentage, treatment content, and treatment procedure represented in examples below can be changed as necessary without departing from the scope of the present invention. Accordingly, the scope of the present invention should not be restricted to the specific examples represented below.

### [Measurements of the average fiber diameter D and the average fiber length L]

The average fiber diameter D and the average fiber length L of the cellulose fibers obtained from Production Examples 1 to 3 were determined using TEM images and SEM images according to the procedures described in paragraph [0020], and the aspect ratio L/D was determined from these values.

### [Production Example 1: Production of microcrystalline cellulose-derived cellulose fibers]

A mixture of 15 parts by mass of commercially available microcrystalline cellulose (manufactured by Funakoshi Co., Ltd., Funacel powder II for column chromatography) and 1,000 parts by mass of purified water was dispersed, and then ground at 200 MPa 300 times with a high pressure grinding machine manufactured by Sugino Machine Limited (Starburst System) to obtain an aqueous dispersion liquid (MC) of microcrystalline cellulose-derived cellulose fibers. The resulting dispersion liquid was measured and placed in a petri dish, and dried at 110 °C for 5 hours to remove water. The amount of the residue was then measured to determine the concentration of the dispersion liquid. As a result, the concentration of cellulose (the concentration of solids) in water was 1.2% by mass.

### [Production Example 2: Production of pulp-derived cellulose fibers]

A mixture of 32.6 parts by mass of a commercially available kraft pulp (manufactured by Kokusai Pulp & Paper Co., Ltd., LBKP D-8, 46% by mass of solids) and 350 parts by mass of purified water was dispersed, and then ground at 245 MPa 315 times with a high pressure grinding machine manufactured by Sugino Machine Limited (Starburst System) to obtain an aqueous dispersion liquid (PC) of pulp-derived cellulose fibers. The resulting dispersion liquid was measured and placed in a petri dish, and dried at 110°C for 5 hours to remove water. The amount of the residue was then measured to determine the concentration of the dispersion liquid. As a result, the concentration of cellulose (the concentration of solids) in water was 1.7% by mass.

### [Production Example 3: Production of pulp-derived cellulose fibers in a polyalcohol aqueous solution]

A mixture of 40 parts by mass of a commercially available kraft pulp (manufactured by Kokusai Pulp & Paper Co., Ltd., LBKP D-8, 46% by mass of solids), 370 parts by mass of purified water, and 100 parts by mass of 1,3-butylene glycol was dispersed, and then ground at 245 MPa 202 times with a high pressure grinding machine manufactured by Sugino Machine Limited (Starburst System) to obtain an aqueous dispersion liquid (PCB) of pulp-derived cellulose fibers. The resulting dispersion liquid was filtrated using a 0.45 micrometer membrane filter, and then washed with 100 parts by mass of purified water twice. Subsequently the resulting sheet-like cellulose fibers were placed in a petri dish, and dried at 110 °C for 2 hours. The resulting film-like dry solid was weighed to determine the concentration of the dispersion liquid. As a result, the concentration of cellulose (the concentration of solids) in water was 2.4% by mass.

The average fiber diameter D and the average fiber length L of the cellulose fibers from Production Examples 1 to 3 were determined according to the above-mentioned procedures. The results obtained and the aspect ratios (L/D) determined from the average fiber diameter D and the average fiber length L are listed in Table 1.

**[Table 1]**

| | Average fiber diameter D [µm] | Average fiber length L [µm] | Aspect ratio L/D |
|---|---|---|---|
| Production Example 1 (MC) | 24 | 594 | 24 |
| Production Example 2 (PC) | 23 | 1930 | 85 |
| Production Example 3 (PCB) | 21 | 1320 | 63 |

### [Production Example 4: Production of pulp-derived cellulose fibers]

2.2 parts by mass of a commercially available kraft pulp (manufactured by Kokusai Pulp & Paper Co., Ltd., LBKP D-8, 46% by mass of solids) was added to 12.8 parts by mass of purified water and dispersed, and processed at 1500 rpm by a contact operation 9 times with a millstone type grinding machine (Super Masscolloider) manufactured by MASUKO SANGYO CO., LTD. with a grinding stone exchanged in the sequence of #16, 46, and 80. The resulting pulp slurry was measured and placed in a petri dish, and dried at 110 °C for 5 hours to remove water. The amount of the residue was then measured to determine the concentration of the dispersion liquid. As a result, the concentration of cellulose (the concentration of solids) in water was 3.5% by mass. Subsequently, the pulp slurry was diluted to 1% by mass with purified water, and placed in a wet type fine grinding machine (NanoVater) manufactured by Yoshida Kikai Co., Ltd., and processed at a nozzle diameter of 0.14 mm and 200 MPa, 50, 100, and 150 times to obtain an aqueous dispersion liquid (PC1, 2, and 3 respectively) of pulp-derived cellulose fibers.

[Production Example 5: Production of pulp-derived cellulose fibers] 1 % by mass of the pulp slurry obtained by using a millstone type grinding machine (Super Masscolloider) manufactured by MASUKO SANGYO CO., LTD. in Production Example 4 was ground at 245 MPa 30, 50, 100, 200, and 300 times with a high pressure grinding machine manufactured by Sugino Machine Limited (Starburst System) to obtain an aqueous dispersion liquid (PC4, 5, 6, 7, and 8, respectively) of pulp-derived cellulose fibers. The dispersion liquid (PC-8) ground 300 times was measured and placed in a petri dish, and dried at 110°C for 5 hours to remove water. The amount of the residue was then measured to determine the concentration of the dispersion liquid. As a result, the concentration of cellulose (the concentration of solids) in water was 0.62% by mass.

### [Example 1 and Comparative Examples 1 to 4]

Test liquids in examples and comparative examples listed in Table 2 were prepared using cellulose fibers prepared in Production Examples 1 and 2 and various thickening components.

First, 1% by mass of an aqueous dispersion liquid or an aqueous solution (0.1% by mass only for a carbomer) was prepared for each of cellulose fibers MC and PC prepared in Production Examples 1 and 2, and general-purpose thickening components (crystalline cellulose), a carbomer, a xanthan gum). Then sodium chloride was added thereto to obtain 0.01% by mass of solutions. Subsequently, these aqueous dispersion liquids or aqueous solutions were adjusted such that these pHs are from 3 to 11 using 0.1 N hydrochloric acid or 0.1 N aqueous sodium hydroxide. These solutions having the total amount of 3 g were placed in screw tubes (manufactured by Maruemu Corporation, No.2) and sealed, and then homogenized by vortexing for 30 seconds. For crystalline cellulose (Comparative Example 2), Cellodene 4M (a content of 4% by mass of cellulose) manufactured by Dai-ichi Kogyo Seiyaku Co., Ltd. was diluted to 1.5% by mass with refined water, and then stirred at 5000 rpm for 1 hour with a homomixer (LR-1A) manufactured by MIZUHO INDUSTRIAL CO., LTD. to obtain a test liquid.

### [States of test liquids]

The test liquids in Example 1 and Comparative Examples 1 to 4 were allowed to stand for 1 day, and then glass containers were turned over. Such test liquids were evaluated through visual determination based on the following criteria. The results are listed in Table 2.

### <Evaluation criteria>

⊙: A test liquid gelated and the surface of the liquid did not fluctuate when vibration was applied.
○: A test liquid gelated but the surface of the liquid fluctuated when vibration was applied.
×: A test liquid did not gelate and exhibited flowability.

**[Table 2]**

| | | | States of test liquids | | | | | | | | |
|---|---|---|---|---|---|---|---|---|---|---|---|
| | | pH | 3 | 4 | 5 | 6 | 7 | 8 | 9 | 10 | 11 |
| Example | 1 | Production Example 2 PC | ⊙ | ⊙ | ○ | ○ | ○ | ○ | ○ | ○ | ○ |
| Comparative Example | 1 | Production Example 1 MC | × | × | × | × | × | × | × | × | × |
| | 2 | Crystalline cellulose | × | - | ○ | - | ○ | - | - | × | × |
| | 3 | Carbomer | × | × | × | ○ | ○ | ○ | × | × | × |
| | 4 | Xanthan gum | × | ○ | ○ | ○ | ○ | ○ | ○ | ○ | × |

Components used in examples and comparative examples listed in Table 2 are as follows:
Cellulose fibers PC: cellulose fibers obtained from Production Example 2
Cellulose fibers MC: cellulose fibers obtained from Production Example 1
Crystalline cellulose: manufactured by Dai-ichi Kogyo Seiyaku Co., Ltd., Cellodene 4M
Carbomer: manufactured by ITO Inc., Carbopol 940
Xanthan gum: manufactured by Sansho Co., Ltd., KETROL CG-SFT

### [Example 2 and Comparative Examples 5 to 8]

Test liquids in examples and comparative examples were prepared using cellulose fibers prepared in Production Examples 1 and 2 and thickening components.

First, a predetermined amount of refined water was added to each of cellulose fibers MC (Comparative Example 5) or PC (Example 2) prepared in Production Examples 1 and 2 and general-purpose thickening components (crystalline cellulose (Comparative Example 6), a carbomer (Comparative Example 7), and a xanthan gum (Comparative Example 8)) such that the concentration of each component was 1% by mass (0.1% by mass only for a carbomer). Then, sodium chloride was added thereto in an amount of 0.01% by mass to 10% by mass with respect to the total amount of the test liquid, and the resultant mixture was homogenized by vortexing for 30 seconds. For a carbomer (Comparative Example 7), the pH of a solution was adjusted to 7 by neutralization using 0.1 N or 1.0 N aqueous sodium hydroxide to obtain a test liquid.

### [Viscosity]

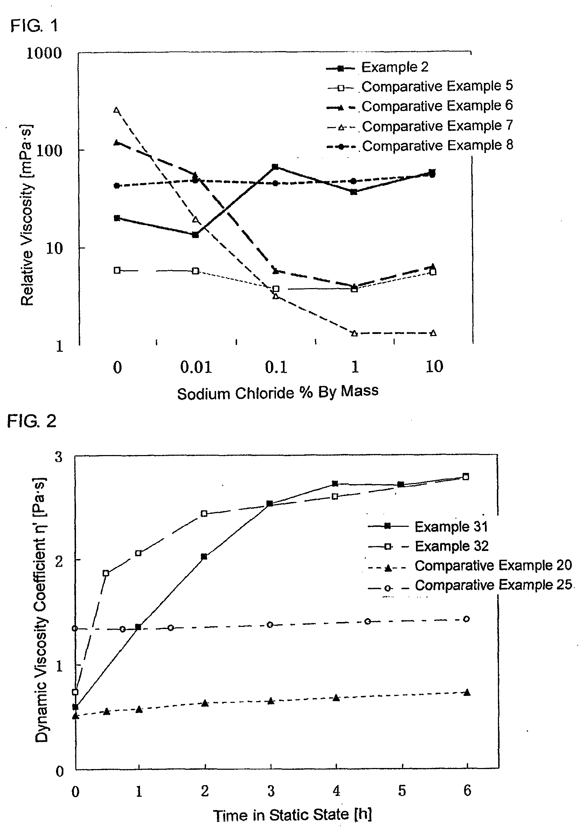

Viscosity measurements with a tuning fork vibration (SV-1A, A & D Company Ltd.) were performed at 25°C for test liquids in Example 2 and Comparative Examples 5 to 8. The results obtained are shown in FIG. 1.

### [Examples 3 to 24 and Comparative Examples 9 to 18]

Test liquids in examples and comparative examples having compositions listed in Table 3 were prepared using cellulose fibers prepared in Production Example 1 or 3 and additives.

First, a predetermined amount of refined water was added to cellulose fibers MC prepared in Production Example 1 such that 1,3-butylene glycol (1,3-BG) was 6.7% by mass with respect to the total amount of the test liquid, or a predetermined amount of refined water was added to cellulose fibers PCB prepared in Production Example 3 such that the cellulose concentration was 0.8% by mass (containing 6.7% by mass of 1,3-BG) with respect to the total amount of the test liquid. The additives listed in Table 3 were added thereto in an amount of 0.01% by mass to 0.1% by mass with respect to the total amount of the test liquid. The resultant mixture having the total amount of 3 g was placed in a screw tube (manufactured by Maruemu Corporation, No.2) and sealed, and then homogenized by vortexing for 30 seconds

### [Visual determination]

The test liquids in Examples 3 to 24 and Comparative Examples 9 to 18 were allowed to stand for 1 day, and then glass containers were turned over. Such test liquids were evaluated through visual determination based on the following criteria. The results are listed in Table 3.

### <Evaluation criteria>

⊙: A test liquid gelated and the surface of the liquid did not fluctuate when vibration was applied.
○: A test liquid gelated but the surface of the liquid fluctuated when vibration was applied.
Δ: A test liquid did not gelate but exhibited decreased flowability.
×: A test liquid did not gelate and exhibited no change in flowability.

**[Table 3]**

| | Production Example 1 MC | Production Example 3 PCB | 1,3-BG | Add itives | Visual determination (gelation) | | | |
|---|---|---|---|---|---|---|---|---|
| | % by mass | % by mass | % by mass | Addition concentration (% by mass) | 0.01 | 0.03 | 0.06 | 0.1 |
| Example 3 | | 0.8 | 6.7 | Sodium chloride | ○ | ⊙ | ⊙ | ⊙ |
| Example 4 | | 0.8 | 6.7 | Potassium chloride | ○ | ⊙ | ⊙ | ⊙ |
| Example 5 | | 0.8 | 6.7 | Zinc chloride | ⊙ | ⊙ | ⊙ | ⊙ |
| Example 6 | | 0.8 | 6.7 | Calcium chloride | ⊙ | ⊙ | ⊙ | ⊙ |
| Example 7 | | 0.8 | 6.7 | Magnesium chloride | ⊙ | ⊙ | ⊙ | ⊙ |
| Example 8 | | 0.8 | 6.7 | Sodium dihydrogenphosphate | Δ | ○ | ⊙ | ⊙ |
| Example 9 | | 0.8 | 6.7 | Sodium hydrogencarbonate | Δ | ○ | ⊙ | ⊙ |
| Example 10 | | 0.8 | 6.7 | Sodium sulfite | ○ | ⊙ | ⊙ | ⊙ |
| Example 11 | | 0.8 | 6.7 | Sodium sulfate | ○ | ⊙ | ○ | ○ |
| Example 12 | | 0.8 | 6.7 | Glucosamine hydrochloride | × | Δ | ⊙ | ⊙ |
| Example 13 | | 0.8 | 6.7 | Dipotassium glycyrrhizinate | × | Δ | ⊙ | ⊙ |
| Example 14 | | 0.8 | 6.7 | Disodium dihydrogen ethylenediamine tetraacetate dihydrate | Δ | ○ | ⊙ | ⊙ |
| Example 15 | | 0.8 | 6.7 | Sodium citrate dihydrate | Δ | ○ | ⊙ | ⊙ |
| Example 16 | | 0.8 | 6.7 | Sodium ascorbate | Δ | ○ | ⊙ | ⊙ |
| Example 17 | | 0.8 | 6.7 | Magnesium L-ascorbyl 2-phosphate | ○ | ⊙ | ⊙ | ⊙ |
| Example 18 | | 0.8 | 6.7 | Sodium lactate (50% aqueous solution) | ○ | ⊙ | ⊙ | ⊙ |
| Example 19 | | 0.8 | 6.7 | Sodium dodecylbenzenesulfonate | × | Δ | Δ | ○ |
| Example 20 | | 0.8 | 6.7 | Sodium lauryl sulfate | × | Δ | ○ | ○ |
| Example 21 | | 0.8 | 6.7 | Lauramidopropyl betaine | × | Δ | ⊙ | ⊙ |
| Example 22 | | 0.8 | 6.7 | Lauryldimethylaminoace tic acid betaine | × | ○ | ⊙ | ⊙ |
| Example 23 | | 0.8 | 6.7 | Lauryl hydroxy sulfobetaine | × | ○ | ⊙ | ⊙ |
| Example 24 | | 0.8 | 6.7 | Lauryl sodium aspartate | Δ | ○ | ⊙ | ⊙ |
| Comparative Example 9 | | 0.8 | 6.7 | Polyoxyethylene oleyl ether | × | × | × | × |
| Comparative Example 10 | | 0.8 | 6.7 | Polyoxyethylene hydrogenated castor oil | × | × | × | × |
| Comparative Example 11 | | 0.8 | 6.7 | Polyoxyethylene Glyceryl Pyroglutamate Iso stearate | × | × | × | × |
| Comparative Example 12 | | 0.8 | 6.7 | Polyoxyethylene sorbitan monostearate | × | × | × | × |
| Comparative Example 13 | | 0.8 | 6.7 | Polyoxyethylene sorbitan monopalmitate | × | × | × | × |
| Comparative Example 14 | 0.8 | | 6.7 | Sodium chloride | × | × | × | × |
| Comparative Example 15 | 0.8 | | 6.7 | Potassium chloride | × | × | × | × |
| Comparative Example 16 | 0.8 | | 6.7 | Zinc chloride | × | × | × | × |
| Comparative Example 17 | 0.8 | | 6.7 | Calcium chloride | × | × | × | × |
| Comparative Example 18 | 0.8 | | 6.7 | Magnesium chloride | × | × | × | × |

Components used in examples and comparative examples shown in Table 3 are as follows:
Sodium chloride: manufactured by Kanto Chemical Co., Inc.
Potassium chloride: manufactured by Kanto Chemical Co., Inc.
Zinc chloride: manufactured by Wako Pure Chemical Industries, Ltd.
Calcium chloride: manufactured by Wako Pure Chemical Industries, Ltd.
Magnesium chloride: manufactured by Wako Pure Chemical Industries, Ltd.
Sodium dihydrogenphosphate: manufactured by JUNSEI CHEMICAL CO., LTD.
Sodium hydrogencarbonate: manufactured by Kanto Chemical Co., Inc.
Sodium sulfite: manufactured by JUNSEI CHEMICAL CO., LTD.
Sodium sulfate: manufactured by JUNSEI CHEMICAL CO., LTD.
Glucosamine hydrochloride: manufactured by Tokyo Chemical Industry Co., Ltd.
Dipotassium glycyrrhizinate: manufactured by PINOA Co., Ltd.
Disodium dihydrogen ethylenediamine tetraacetate dihydrate: manufactured by JUNSEI CHEMICAL CO., LTD.
Sodium citrate dihydrate: manufactured by Kanto Chemical Co., Inc.
Sodium ascorbate: manufactured by JUNSEI CHEMICAL CO., LTD.
Magnesium L-ascorbyl 2-phosphate: manufactured by ITO Inc., APM
Sodium lactate (50% aqueous solution): manufactured by JUNSEI CHEMICAL CO., LTD.
Sodium dodecylbenzenesulfonate: manufactured by Kanto Chemical Co., Inc.
Sodium lauryl sulfate: manufactured by JUNSEI CHEMICAL CO., LTD.
Lauramidopropyl betaine: manufactured by Kao Corporation, Amphitol 20AB
Lauryldimethylaminoacetic acid betaine: manufactured by Kao Corporation, Amphitol 20HD
Lauryl hydroxy sulfobetaine: manufactured by Kao Corporation, Amphitol 20BS
Lauryl sodium aspartate: manufactured by Asahi Kasei Chemicals Corporation, AminoFormer FLDS-L
Polyoxyethylene oleyl ether: manufactured by TOHO Chemical Industry Co., Ltd., Pegnol O-20
Polyoxyethylene hydrogenated castor oil: manufactured by Nikko Chemicals Co., Ltd., HCO-60
Polyoxyethylene Glyceryl Pyroglutamate Isostearate: manufactured by NIHON EMULSION Co., Ltd., PYROTER GPI-25
Polyoxyethylene sorbitan monostearate: manufactured by Kanto Chemical Co., Inc., Tween 60
Polyoxyethylene sorbitan monopalmitate: manufactured by Kanto Chemical Co., Inc., Tween 40

### [Examples 25 to 30 and Comparative Example 19]

Test liquids in examples and comparative examples having compositions listed in Table 4 were prepared using cellulose fibers PC prepared in Production Example 2 and additives.

First, a predetermined amount of refined water was added to cellulose fibers PC prepared in Production Example 3 such that the cellulose concentration was 1.0% by mass. Then, additives listed in Table 4 were added thereto in an amount of 0.01% by mass to 0.1% by mass with respect to the total amount of the test liquid. The resultant mixture having the total amount of 3 g was placed in a screw tube (manufactured by Maruemu Corporation, No.2) and sealed, and then homogenized by vortexing for 30 seconds.

### [Visual determination]

The test liquids in Examples 25 to 30 and Comparative Example 19 were allowed to stand for 1 day, and then glass containers were turned over. Such test liquids were evaluated through visual determination based on the following criteria. The results are listed in Table 4.

### <Evaluation criteria>

⊙: A test liquid gelated and the surface of the liquid did not fluctuate when vibration was applied.
○: A test liquid gelated but the surface of the liquid fluctuated when vibration was applied.
Δ: A test liquid did not gelate but exhibited decreased flowability.
×: A test liquid did not gelate and exhibited no change in flowability.

**[Table 4]**

| | Production Example 2 PC | Additives | Visual determination (gelation) | |
|---|---|---|---|---|
| | % by mass | Addition concentration (% by mass) | 0.01 | 0.1 |
| Example 25 | 1 | Hypromellose | Δ | ○ |
| Example 26 | 1 | Polyacrylic acid | Δ | ○ |
| Example 27 | 1 | Chitosan | ⊙ | * |
| Example 28 | 1 | Propylene glycol alginate | Δ | ○ |
| Example 29 | 1 | Xanthan gum | Δ | ○ |
| Example 30 | 1 | Carboxymethylcellulose | × | ○ |
| Comparative Example 19 | 1 | Hydroxypropylcellulose | × | × |

| | | | | |
|---|---|---|---|---|
| * Aggregation occurs, resulting in decreased viscosity. | | | | |

Components used in examples and comparative examples shown in Table 4 are as follows:
Hypromellose: manufactured by Shin-Etsu Chemical Co., Ltd., Metolose 60SH
Polyacrylic acid: manufactured by Aldrich
Chitosan: manufactured by ACROS
Propylene glycol alginate: manufactured by KIMICA Corporation, Kimiloid HV
Xanthan gum: manufactured by Sansho Co., Ltd., KETROL CG-SFT
Carboxymethylcellulose: manufactured by AS ONE Corporation
Hydroxypropylcellulose: manufactured by NIPPON SODA CO., LTD., HPC SSL

### [Examples 31 and 32 and Comparative Examples 20 to 25]

Test liquids in examples and comparative examples having compositions listed in Table 5 were prepared using cellulose fibers prepared in Production Examples 1 and 2 and thickening components.

First, to produce compositions listed in Table 5, cellulose fibers MC or PC prepared in Production Example 1 or 2, or general-purpose thickening components (crystalline cellulose, a carbomer, a xanthan gum), a polyalcohol and sodium lactate as a moisturizer, ethanol as a refrigerant, and refined water were blended. The resultant mixture having the total amount of 3 g was placed in a screw tube (manufactured by Maruemu Corporation, No.2) and sealed, and then homogenized by vortexing for 30 seconds. For crystalline cellulose (Comparative Example 23), Cellodene 4M (a content of 4% by mass of cellulose) manufactured by Dai-ichi Kogyo Seiyaku Co., Ltd. was diluted to 1.5% by mass with refined water, and then stirred at 5000 rpm for 1 hour with a homomixer (LR-1A) manufactured with MIZUHO INDUSTRIAL CO., LTD. to obtain a test liquid. For a carbomer (Comparative Example 24), the carbomer was dissolved into a polyalcohol and ion exchange water, and then the resultant mixture was neutralized using 0.1 N or 1.0 N aqueous sodium hydroxide to obtain a test liquid.

In addition, the test liquid prepared in Example 31 was allowed to stand over night to be gelated, and then stirred vigorously by vortexing for 30 seconds to obtain a test liquid in the state of sol for Example 32.

### [Visual determination]

The test liquids in Example 31 and Comparative Examples 20 to 25 were allowed to stand for 1 day, and then glass containers were turned over. Such test liquids were evaluated through visual determination based on the following criteria. The results are listed in Table 5.

### <Evaluation criteria>

⊙: A test liquid gelated and the surface of the liquid did not fluctuate when vibration was applied.
○: A test liquid gelated but the surface of the liquid fluctuated when vibration was applied.
Δ: A test liquid did not gelate but exhibited decreased flowability.
×: A test liquid did not gelate and exhibited no change in flowability.

### [Viscosity]

Viscosity measurements with a tuning fork vibration (SV-1A, A & D Company Ltd.) were performed at 25°C for test liquids in Example 31 and Comparative Examples 20 to 25. The results are listed in Table 5.

### [Spray testing]

The test liquids in Example 31 and Comparative Examples 20 to 25 were poured into spray vials (manufactured by Maruemu Corporation, No.2), and sprayed onto glass substrates from a distance of 3.5 cm once. The spread of mist attached to the glass substrates was evaluated based on the following evaluation criteria. The results are listed in Table 5.

### <Evaluation criteria>

### Spread of mist

○: A diameter of 4 to 2 cm in full-cone type (entire circular area) spraying
×: A diameter of 2 cm or less in solid type (straight advancing type) spraying, or incapable of spraying

Components used in examples and comparative examples listed in Table 5 are as follows:
Cellulose fibers PC: cellulose fibers obtained from Production Example 2
Cellulose fibers MC: cellulose fibers obtained from Production Example 1
Crystalline cellulose: manufactured by Dai-ichi Kogyo Seiyaku Co., Ltd., Cellodene 4M
Carbomer: manufactured by ITO Inc., Carbopol 940
Xanthan gum: manufactured by Sansho Co., Ltd., KETROL CG-SFT
1,3-BG: manufactured by ITO Inc., 1,3-butylene glycol
Sodium lactate: manufactured by JUNSEI CHEMICAL CO.,LTD., Japanese Standards of Quasi-drug Ingredients, sodium lactate solution 50%
Ethanol: manufactured by Kanto Chemical Co., Inc., ethanol

### [Thixotropy evaluation]

Each of the test liquids right after preparation in Examples 31 and 32 and Comparative Examples 20 to 25 was placed in a sample chamber of the rheometer "Rheosol-G1000 model" manufactured by UBM. Then, dynamic viscosity coefficients η' were recorded at a predetermined time interval in a cone plate with a sealing hood attached for preventing concentration variations of test liquids at 25°C at a dynamic displacement of 0.5 deg with a rotor vibrated at a frequency of 1Hz. The vibration of the rotor was stopped and the samples were in a static state during measurements. The results obtained are shown in both Table 6 and FIG. 2.

**[Table 5]**

| | | Example | Comparative Examples | | | | | |
|---|---|---|---|---|---|---|---|---|
| | | 31 | 20 | 21 | 22 | 23 | 24 | 25 |
| Formulation | PC | 1 | 1 | | | | | |
| | MC | | | 1 | | | | |
| | Crystalline cellulose | | | | 1 | 1* | | |
| | Carbomer | | | | | | 0.1 | |
| | Xanthan gum | | | | | | | 1 |
| | 1,3-BG | 5 | 5 | 5 | 5 | 5 | 5 | 5 |
| | Sodium lactate | 0.01 | | 0.01 | 0.01 | 0.01 | 0.01 | 0.01 |
| | Ethanol | 5 | 5 | 5 | 5 | 5 | 5 | 5 |
| | Refined water | 89.0 | 89.0 | 89.0 | 89.0 | 89.0 | 89.9 | 89.0 |
| Visual determination | Gelation | ○ | × | × | × | ○ | × | ○ |
| Viscosity | mPa/s | 12 | 17 | 12 | 4.5 | 106 | 36 | 40 |
| Spray testing | Spread of mist | ○ | ○ | ○ | ○ | ○ | × | × |

| | | | | | | | | |
|---|---|---|---|---|---|---|---|---|
| *A component obtained by diluting Cellodene to 1.5% by mass and stirring the resultant mixture with a homomixer at 5000 rpm for 1 hour was used. | | | | | | | | |

**[Table 6]**

| | η' %/0h | | | |
|---|---|---|---|---|
| Time (h) | Example 31 | Example 32 | Comparative Example 20 | Comparative Example 25 |
| 0 | 100.0 | 100.0 | 100.0 | 100.0 |
| 0.5 | - | 252.7 | 108.7 | - |
| 0.75 | - | - | - | 99.3 |
| 1 | 225.8 | 278.5 | 112.0 | - |
| 1.5 | - | - | - | 100.0 |
| 2 | 337.8 | 330.2 | 121.8 | - |
| 3 | 423.1 | - | 126.1 | 102.2 |
| 4 | 454.8 | 351.9 | 131.1 | - |
| 4.5 | - | - | - | 104.5 |
| 5 | 453.2 | - | - | - |
| 6 | 464.9 | 376.4 | 139.8 | 106.0 |

### [Example 33 and Comparative Examples 26 to 30]

The test liquids in examples and comparative examples having compositions listed in Table 7 were prepared.

First, to produce compositions listed in Table 7, Pegnol O-20 was added to an olive oil, and the resultant mixture was homogenized by vortexing for 30 seconds. Then, 1,3-butylene glycol, sodium lactate, cellulose fibers MC or PC prepared in Production Example 1 or 2 as an emulsion stability component, and refined water were added thereto. The resultant mixture having the total amount of 3 g was placed in a screw tube (manufactured by Maruemu Corporation, No.2) and sealed, and then homogenized by vortexing for 30 seconds. For comparison, test liquids were prepared using general-purpose thickening components (crystalline cellulose, a carbomer, a xanthan gum) listed in Table 7 in the same manner as the example. For crystalline cellulose (Comparative Example 27), Cellodene 4M (a content of 4% by mass of cellulose) manufactured by Dai-ichi Kogyo Seiyaku Co., Ltd. was diluted to 1.5% by mass with refined water, and then stirred at 5000 rpm for 1 hour with a homomixer (LR-1A) manufactured by MIZUHO INDUSTRIAL CO., LTD. to obtain a test liquid. For a carboxyvinyl polymer (carbomer, Comparative eEample 29), after dissolution of the carboxyvinyl polymer, the resultant mixture was neutralized using 1.0 N aqueous sodium hydroxide for use.

### [Visual determination]

The test liquids in Example 33 and Comparative Examples 26 to 30 were allowed to stand for 1 day, and then glass containers were turned over. Such test liquids were evaluated through visual determination based on the following criteria.

### <Evaluation criteria>

⊙: A test liquid gelated and the surface of the liquid did not fluctuate when vibration was applied.
○: A test liquid gelated but the surface of the liquid fluctuated when vibration was applied.
Δ: A test liquid did not gelate but exhibited decreased flowability.
×: A test liquid did not gelate and exhibited no change in flowability.

### [Viscosity]

Viscosity measurements with a tuning fork vibration (SV-1A, A & D Company Ltd.) were performed at 25°C for test liquids in Example 33 and Comparative Examples 26 to 30.

### [Appearance of emulsified liquids]

Temporal stability (homogeneity) of test liquids in Example 33 and Comparative Examples 26 to 30 stored in a thermostatic oven at 50°C for 1 month was evaluated through visual determination based on the following criteria.

### <Evaluation criteria>

### Homogeneity

○: Gelation was observed with no change at all in appearance, and no syneresis and separation were observed.
Δ: No change in appearance, and no syneresis and separation were observed, but flowability was observed.
×: Syneresis and separation were observed.
The results of the evaluation are listed in Table 7.

Components used in Table 7 are as follows:
Cellulose fibers PC: cellulose obtained fibers from Production Example 2
Cellulose fibers MC: cellulose fibers obtained from Production Example 1
Crystalline cellulose: manufactured by Dai-ichi Kogyo Seiyaku Co., Ltd., Cellodene 4M
Carbomer: manufactured by ITO Inc., Carbopol 940
Xanthan gum: manufactured by Sansho Co., Ltd., KETROL CG-SFT
1,3-BG: manufactured by ITO Inc., 1,3-butylene glycol
Olive oil: manufactured by JUNSEI CHEMICAL CO., LTD., Japanese Standards of Quasi-drug Ingredients, olive oil
Pegnol: manufactured by TOHO Chemical Industry Co., Ltd., Pegnol O-20
Sodium lactate: manufactured by JUNSEI CHEMICAL CO., LTD., Japanese Standards of Quasi-drug Ingredients, sodium lactate solution 50%

**[Table 7]**

| | | Example | Comparative Examples | | | | |
|---|---|---|---|---|---|---|---|
| | | 33 | 26 | 27 | 28 | 29 | 30 |
| Formulation | PC | 1 | | | | | |
| | MC | | 1 | | | | |
| | Crystalline cellulose | | | 1 | 1* | | |
| | Carbomer | | | | | 0.1 | |
| | Xanthan gum | | | | | | 1 |
| | 1,3-BG | 5 | 5 | 5 | 5 | 5 | 5 |
| | Sodium | 0.01 | 0.01 | 0.01 | 0.01 | 0.01 | 0.01 |
| | lactate | | | | | | |
| | Pegnol O-20 | 0.1 | 0.1 | 0.1 | 0.1 | 0.1 | 0.1 |
| | Olive oil | 10 | 10 | 10 | 10 | 10 | 10 |
| | Refined water | 83.9 | 83.9 | 83.9 | 83.9 | 84.8 | 83.9 |
| Visual determination | Gelation | ○ | × | × | ○ | Δ | ○ |
| Relative viscosity | mPa/s | 19 | 16 | 6 | 59 | 54 | 52 |
| Appearance of emulsified liquids | 50°C, 1 month | ○ | Δ | × | × | Δ | × |

| | | | | | | | |
|---|---|---|---|---|---|---|---|
| *A component obtained by diluting Cellodene to 1.5% by mass and stirring the resultant mixture with a homomixer at 5000 rpm for 1 hour was used. | | | | | | | |

### [Examples 34 to 36]

Test liquids in examples having compositions listed in Table 8 were prepared using cellulose fibers prepared in Production Example 4. 1 part by mass of 1 % by mass of an aqueous solution of sodium lactate was added to 100 parts by mass of an aqueous dispersion liquid of cellulose fibers, and the resultant mixture was homogenized by vortexing for 30 seconds to produce a test liquid.

### [Examples 37 to 46]

Test liquids in examples having compositions listed in Table 9 were prepared using cellulose fibers prepared in Production Example 5. 0.5 by mass or 0.6 part by mass of 1 % by mass of an aqueous solution of sodium lactate was added to 100 parts by mass of 0.5% by mass or 0.6% by mass of an aqueous dispersion liquid of cellulose fibers homogenized by addition of purified water, and the resultant mixture was homogenized by vortexing to produce a test liquid.

### [Visual determination]

The test liquids in Examples 34 to 46 were allowed to stand for 1 day, and then glass containers were turned over. Such test liquids were evaluated through visual determination based on the following criteria. The results of the evaluation are listed in Tables 8 and 9.

### <Evaluation criteria>

⊙: A test liquid gelated and the surface of the liquid fluctuated when vibration was applied.
○: A test liquid gelated but the surface of the liquid fluctuated when vibration was applied.
Δ: A test liquid did not gelate but exhibited decreased flowability.
×: A test liquid did not gelate and exhibited no change in flowability.

**[Table 8]**

| | Production Example 4 | | | Sodium lactate | Gelation |
|---|---|---|---|---|---|
| | PC1 | PC2 | PC3 | | |
| The number of processes | 50 | 100 | 150 | | |
| Example 34 | 1 | | | 0.01 | ⊙ |
| Example 35 | | 1 | | 0.01 | ⊙ |
| Example 36 | | | 1 | 0.01 | ⊙ |

| | | | | | |
|---|---|---|---|---|---|
| *All units are represented by % by mass in test liquids. | | | | | |

**[Table 9]**

| | Production Example 5 | | | | | Sodium lactate | Gelation |
|---|---|---|---|---|---|---|---|
| | PC4 | PC5 | PC6 | PC7 | PC8 | | |
| The number of processes | 30 | 50 | 100 | 200 | 300 | | |
| Example 37 | 0.6 | | | | | 0.006 | ⊙ |
| Example 38 | 0.5 | | | | | 0.005 | ○ |
| Example 39 | | 0.6 | | | | 0.006 | ○ |
| Example 40 | | 0.5 | | | | 0.005 | ○ |
| Example 41 | | | 0.6 | | | 0.006 | ○ |
| Example 42 | | | 0.5 | | | 0.005 | ○ |
| Example 43 | | | | 0.6 | | 0.006 | ○ |
| Example 44 | | | | 0.5 | | 0.005 | ○ |
| Example 45 | | | | | 0.6 | 0.006 | ○ |
| Example 46 | | | | | 0.5 | 0.005 | ○ |

| | | | | | | | |
|---|---|---|---|---|---|---|---|
| *All units are represented by % by mass in test liquids. | | | | | | | |

As is evident from the results listed in Table 2, in Example 1 in which cellulose fibers (PC) obtained from Production Example 2 of the present invention were added, thickening properties were observed in any pH of a test liquid, and a gel was formed. In Comparative Examples 2 to 4 in which cellulose fibers (MC) obtained from Production Example 1 and thickening components including crystalline cellulose, a carbomer, or a xanthan gum were added, there was no thickening effect in any pH of test liquids, and flowability was observed. Further, only a region of the test liquid around which pH was neutral gelated, and flowability was observed in acidic or basic regions, resulting in low stability of thickening performance for pH variations of the test liquids.

As is evident from the results shown in FIG 1, in Example 2 in which cellulose fibers (PC) obtained from Production Example 2 of the present invention were added, an increase in viscosity was observed with increasing concentration of sodium chloride of a test liquid, and the same viscosity as a salt resistant xanthan gum was obtained, resulting in stable thickening performance at a concentration of 0.1% by mass to 10% by mass of sodium chloride. In Comparative Examples 5 to 8 in which cellulose fibers (MC) obtained from Production Example 1 and thickening components including crystalline cellulose and a carbomer were added, there was no thickening effect in any concentration of sodium chloride in test liquids, and flowability was observed. Further, rapid decrease in viscosity was observed with increasing concentration of sodium chloride, resulting in low stability of thickening performance for the concentration of sodium chloride.

As is evident from the results listed in Table 3, in Examples 3 to 24 in which cellulose fibers (PCB) obtained from Production Example 3 of the present invention were added, an increase in viscosity of test liquids was observed with increasing concentration of a variety of electrolyte additives, resulting in formation of gels. On the other hand, in Comparative Examples 9 to 13 in which nonelectrolyte additives were added, there was no change in flowability of test liquids in any concentration of the additives. In Comparative Examples 14 to 18 in which cellulose fibers (MC) obtained from Production Example 1 were added, there was no change in flowability of test liquids even when electrolyte additives were added thereto, and as a result, it was difficult to control thickening performance and gelation with additives.

As is evident from the results listed in Table 4, in Examples 25 to 30 in which cellulose fibers (PC) obtained from Production Example 2 of the present invention were added, an increase in viscosity of test liquids was observed with increasing concentration of a variety of high molecular weight electrolyte additives, resulting in formation of gels. In contrast, in Comparative Example 19 in which a high molecular weight nonelectrolyte additive was added, there was no change in flowability of a test liquid in any concentration of the additive.

As is evident from the results listed in Table 6 and FIG 2, in Example 31 in which cellulose fibers (PC) obtained from Production Example 2 of the present invention were added, a dynamic viscosity coefficient was increased with time right after preparation of a test liquid. The test liquid in Example 32, which was obtained by leaving the test liquid in Example 31 overnight to be gelated, and then stirring the resultant mixture vigorously, also exhibited an increase in a dynamic viscosity coefficient with time. The test liquid in Example 31 exhibited thixotropy, which caused changes from a liquid form through a gel form repeatedly. In contrast, in Comparative Example 20 in which no electrolyte was added, a dynamic viscosity coefficient did not change with time, which was constant, and no thixotropy and no likelihood of gelation were observed. In Comparative Example 25 in which a xanthan gum was added, a dynamic viscosity coefficient did not change with time, which was constant, and no thixotropy was observed.

As is evident from the results listed in Table 5, in Example 31 in which cellulose fibers (PC) obtained from Production Example 2 of the present invention were added, gelation performance and thixotropy were observed while maintaining low viscosity, and also excellent spraying properties were obtained. In contrast, in Comparative Example 21 in which cellulose fibers (MC) obtained from Production Example 1 were added and Comparative Example 22in which crystalline cellulose was added, viscosity of test liquids was low, and no gelation was formed. As a result, it was difficult to control thickening performance and gelation. In Comparative Example 23 in which crystalline cellulose subjected to dispersive treatment with a homomixer was added, a significant increase in viscosity of a test liquid was observed, resulting in formation of gels and sprayability. However, this required special dispersive treatment, leading to lack of versatility. In Comparative Examples 24 and 25 in which general-purpose thickening components including a carbomer and a xanthan gum were added, high viscosity was observed, but flowability was also observed. Therefore, this leads to low sprayability and difficulty in handling.

As is evident from the results listed in Table 7, in Example 33 in which cellulose fibers (PC) obtained from Production Example 2 of the present invention were added, gelation performance and thixotropy were observed while maintaining low viscosity, and excellent emulsion stability was also obtained. In contrast, in Comparative Example 26 in which cellulose fibers (MC) obtained from Production Example 1 were added and Comparative Example 27 in which crystalline cellulose was added, viscosity of test liquids was low, and no gelation was observed, resulting in low emulsion stability. In Comparative Example 28 in which crystalline cellulose subjected to dispersive treatment with a homomixer was added, a significant increase in viscosity of a test liquid was observed and a gel was formed, but syneresis occurred in emulsion, whereby emulsion stability was low. Further, special dispersive treatment was required, resulting in lack of versatility. In Comparative Examples 29 and 30 in which general-purpose thickening components including a carbomer and a xanthan gum were added, viscosity was high, but flowability was also observed, resulting in a concern on long-term stability of emulsion.

As is evident from the results listed in Tables 8 and 9, all test liquids in Examples 34 to 46 including sodium lactate and cellulose fibers (PC1 to PC8) subjected to wet micronization and high pressure grinding at different number of times, which were obtained from Production Example 4 or 5 of the present invention, exhibited good gelatin performance.

### INDUSTRIAL APPLICABILITY

A thickening composition of the present invention can provide a thickening composition that, when added, does not exhibit a significant increase in thickening, and that exhibits gel-forming ability with a variety of electrolyte additives while maintaining low viscosity, gel-forming ability in any pH of a solution, sprayability, and excellent emulsion stability, as well as cosmetics containing the same, and can provide external preparations, skin protective agents, and wound dressings having excellent feel of use.

## Claims

1. A thickening composition comprising:
an electrolyte; and
cellulose fibers having an average fiber diameter (D) of 0.001 to 100 µm and containing hemicellulose and amorphous regions,
**characterized in that** a ratio (L/D) of an average fiber length (L) to the average fiber diameter (D) is 5 to 500, and **in that** the cellulose is derived from broadleaf trees.

2. The thickening composition according to claim 1, wherein the electrolyte is one or two or more selected from the group consisting of sodium chloride, potassium chloride, zinc chloride, calcium chloride, magnesium chloride, sodium dihydrogenphosphate, sodium hydrogencarbonate, sodium sulfite, sodium sulfate, glucosamine hydrochloride, dipotassium glycyrrhizinate, disodium dihydrogen ethylenediamine tetraacetate dihydrate, sodium citrate dihydrate, sodium ascorbate, magnesium L-ascorbyl 2-phosphate, sodium lactate, sodium dodecylbenzenesulfonate, sodium lauryl sulfate, lauramidopropyl betaine, lauryldimethylaminoacetic acid betaine, lauryl hydroxy sulfobetaine, lauryl sodium aspartate, hypromellose, carbomers, polyacrylic acids, chitosans, xanthan gums, propylene glycol alginate, carboxymethylcellulose, and salts thereof.

3. The thickening composition according to a claim 1 or claim 2, comprising the cellulose fibers having an average fiber diameter (D) of 0.001 to 0.05 µm and a ratio (L/D) of an average fiber length (L) to the average fiber diameter (D) of 5 to 500.

4. The thickening composition according to claim 3, **characterized in that** the cellulose fibers are cellulose processed by kraft pulping.

5. The thickening composition according to claim 4, **characterized in that** the cellulose fibers are kraft pulp.

6. The thickening composition according to any one of claims 1 to 5, comprising a proportion of 0.0001 part by mass to 30 parts by mass of the electrolyte with respect to 1 part by mass of the cellulose fibers.

7. Use of the thickening composition according to any one of claims 1 to 6, wherein the thickening composition as an additive added to increase viscosity of a cosmetic.

8. The use according to claim 7, **characterized in that** the thickening composition is an additive added to a cosmetic of a cosmetic product in which the cosmetic is sprayed in use.

9. The thickening composition according to any one of claims 1 to 6, wherein the electrolyte is sodium lactate and the thickening composition exhibits thixotropy.

10. The thickening composition according to any one of claims 1 to 6 and 9, wherein the thickening composition containing 1% by mass of cellulose fibers and 0.01% by mass of the electrolyte, **characterized in that** in dynamic viscoelasticity measurements using a thickening composition containing 1% by mass of cellulose fibers and 0.01% by mass of an electrolyte, a dynamic viscosity coefficient η' measured in a cone plate at a dynamic displacement of 0.5 deg and a frequency of 1Hz in a static state at 25°C for 2 hours is at least 150% of η' at 25°C for 0 hour.

11. A cosmetic comprising the thickening composition according to any one of claims 1 to 6 and 9 to 10.

12. The cosmetic according to claim 11, further comprising an aqueous component.

13. The cosmetic according to claim 11 or 12, further comprising a proportion of 1 part by mass to 100 parts by mass of one or two or more polyalcohols selected from the group consisting of 1,3-butylene glycol, glycerol, and diglycerol with respect to 1 part by mass of the cellulose fibers.

14. The cosmetic according to any one of claims 11 to 13, further comprising an oil component.

15. The cosmetic according to any one of claims 11 to 14, further comprising a surfactant.

16. The cosmetic according to claim 14 or 15, wherein the cosmetic is in a form of emulsion.

17. An external preparation comprising the thickening composition according to any one of claims 1 to 6 and 9 to 10.

## Patentansprüche

1. Verdickungszusammensetzung, umfassend:
einen Elektrolyten; und
Cellulosefasern, die einen durchschnittlichen Faserdurchmesser (D) von 0,001 bis 100 µm aufweisen und Hemicellulose und amorphe Regionen enthalten,
**dadurch gekennzeichnet, dass** das Verhältnis (L/D) der durchschnittlichen Faserlänge (L) zum durchschnittlichen Faserdurchmesser (D) 5 bis 500 beträgt und die Cellulose von Laubbäumen stammt.

2. Verdickungszusammensetzung gemäss Anspruch 1, wobei der Elektrolyt einer oder zwei oder mehr, ausgewählt aus der Gruppe bestehend aus Natriumchlorid, Kaliumchlorid, Zinkchlorid, Calciumchlorid, Magnesiumchlorid, Natriumdihydrogenphosphat, Natriumhydrogencarbonat, Natriumsulfit, Natriumsulfat, Glucosamin-Hydrochlorid, Dikaliumglycyrrhizinat, Dinatriumdihydrogenethylendiamintetraacetatdihydrat, Natriumcitratdihydrat, Natriumascorbat, Magnesium-L-ascorbyl-2-phosphat, Natriumlactat, Natriumdodecylbenzolsulfonat, Natriumlaurylsulfat, Lauramidopropylbetain, Lauryldimethylaminoessigsäurebetain, Laurylhydroxysulfobetain, Laurylnatriumaspartat, Hypromellose, Carbomere, Polyacrylsäuren, Chitosane, Xanthangummen, Propylenglykolalginat, Carboxymethylcellulose und Salze davon, ist.

3. Verdickungszusammensetzung gemäss Anspruch 1 oder Anspruch 2, umfassend Cellulosefasern mit einem durchschnittlichen Faserdurchmesser (D) von 0,001 bis 0,05 µm und einem Verhältnis (L/D) der durchschnittlichen Faserlänge (L) zum durchschnittlichen Faserdurchmesser (D) von 5 bis 500.

4. Verdickungszusammensetzung gemäss Anspruch 3, **dadurch gekennzeichnet, dass** die Cellulosefasern durch Kraftaufschluss verarbeitete Cellulose sind.

5. Verdickungszusammensetzung gemäss Anspruch 4, **dadurch gekennzeichnet, dass** die Cellulosefasern Kraftzellstoff sind.

6. Verdickungszusammensetzung gemäss irgendeinem der Ansprüche 1 bis 5, umfassend einen Anteil von 0,0001 bis 30 Masseteilen des Elektrolyten, bezogen auf 1 Masseteil der Cellulosefasern.

7. Verwendung der Verdickungszusammensetzung gemäss irgendeinem der Ansprüche 1 bis 6, wobei die Verdickungszusammensetzung als Additiv zugegeben wird, um die Viskosität eines Kosmetikums zu erhöhen.

8. Verwendung gemäss Anspruch 7, **dadurch gekennzeichnet, dass** die Verdickungszusammensetzung ein Additiv ist, das zu einem Kosmetikum eines kosmetischen Produkts zugegeben wird, wobei das Kosmetikum bei der Anwendung gesprüht wird.

9. Verdickungszusammensetzung gemäss irgendeinem der Ansprüche 1 bis 6, wobei der Elektrolyt Natriumlactat ist und die Verdickungszusammensetzung Thixotropie aufweist.

10. Verdickungszusammensetzung gemäss irgendeinem der Ansprüche 1 bis 6 und 9, wobei die Verdickungszusammensetzung, enthaltend 1 Masse-% Cellulosefasern und 0,01 Masse-% des Elektrolyten, **dadurch gekennzeichnet ist, dass** bei dynamischen Viskoelastizitätsmessungen unter Verwendung einer Verdickungszusammensetzung, enthaltend 1 Masse-% Cellulosefasern und 0,01 Masse-% eines Elektrolyten, ein dynamischer Viskositätskoeffizient η', der in einer Kegel/Platte bei einer dynamischen Verschiebung von 0,5 Grad und einer Frequenz von 1 Hz in einem statischen Zustand bei 25°C für 2 Stunden gemessen wird, mindestens 150 % von η' bei 25°C für 0 Stunden beträgt.

11. Kosmetikum, das die Verdickungszusammensetzung gemäss irgendeinem der Ansprüche 1 bis 6 und 9 bis 10 umfasst.

12. Kosmetikum gemäss Anspruch 11, das ferner eine wässrige Komponente umfasst.

13. Kosmetikum gemäss Anspruch 11 oder 12, das ferner einen Anteil von 1 bis 100 Masseteilen an einem oder zwei oder mehr Polyalkoholen, ausgewählt aus der Gruppe bestehend aus 1,3-Butylenglykol, Glycerin, und Diglycerin, bezogen auf 1 Masseteil der Cellulosefasern, umfasst.

14. Kosmetikum gemäss irgendeinem der Ansprüche 11 bis 13, das ferner eine Ölkomponente umfasst.

15. Kosmetikum gemäss irgendeinem der Ansprüche 11 bis 14, das ferner ein Tensid umfasst.

16. Kosmetikum gemäss Anspruch 14 oder 15, wobei das Kosmetikum in Form einer Emulsion vorliegt.

17. Zubereitung zur äusseren Anwendung, die die Verdickungszusammensetzung gemäss irgendeinem der Ansprüche 1 bis 6 und 9 bis 10 umfasst.

## Revendications

1. Composition épaississante comprenant :
un électrolyte ;
des fibres de cellulose ayant un diamètre de fibre moyen (D) de 0,001 à 100 µm et contenant de l'hémicellulose et des régions amorphes,
**caractérisée en ce qu'**un rapport (L/D) d'une longueur de fibre moyenne (L) sur le diamètre de fibre moyen (D) est de 5 à 500, et **en ce que** la cellulose est tirée d'arbres à feuilles larges.

2. Composition épaississante selon la revendication 1, dans laquelle l'électrolyte est formé d'un ou deux ou plus choisis dans le groupe constitué du chlorure de sodium, du chlorure de potassium, du chlorure de zinc, du chlorure de calcium, du chlorure de magnésium, du dihydrogénophosphate de sodium, de l'hydrogénocarbonate de sodium, du sulfite de sodium, du sulfate de sodium, du chlorhydrate de glucosamine, du glycyrrhizinate dipotassique, du dihydrogénoéthylènediaminetétraacétate disodique dihydraté, du citrate de sodium dihydraté, de l'ascorbate de sodium, du L-ascorbyl-2-phosphate de magnésium, du lactate de sodium, du dodécylbenzènesulfonate de sodium, du laurylsulfate de sodium, de la lauramidopropylbétaïne, de la bétaïne d'acide lauryldiméthylaminoacétique, de la laurylhydroxysulfobétaïne, du laurylaspartate de sodium, de l'hypromellose, des carbomères, des acides polyacryliques, des chitosanes, des gommes xanthane, de l'alginate de propylèneglycol, de la carboxyméthylcellulose et de leurs sels.

3. Composition épaississante selon la revendication 1 ou la revendication 2, comprenant les fibres de cellulose ayant un diamètre de fibre moyen (D) de 0,001 à 0,05 µm et un rapport (L/D) d'une longueur de fibre moyenne (L) sur le diamètre de fibre moyen (D) de 5 à 500.

4. Composition épaississante selon la revendication 3, **caractérisée en ce que** les fibres de cellulose sont formées de cellulose traitée par production de pâte kraft.

5. Composition épaississante selon la revendication 4, **caractérisée en ce que** les fibres de cellulose sont formées de pâte kraft.

6. Composition épaississante selon l'une quelconque des revendications 1 à 5, comprenant une proportion de 0,0001 partie en masse à 30 parties en masse de l'électrolyte par rapport à 1 partie en masse des fibres de cellulose.

7. Utilisation de la composition épaississante selon l'une quelconque des revendications 1 à 6, dans laquelle la composition épaississante est un additif ajouté pour augmenter la viscosité d'un cosmétique.

8. Utilisation selon la revendication 7, **caractérisée en ce que** la composition épaississante est un additif ajouté à un cosmétique d'un produit cosmétique dans lequel le cosmétique est pulvérisé au cours de son utilisation.

9. Composition épaississante selon l'une quelconque des revendications 1 à 6, dans laquelle l'électrolyte est le lactate de sodium et la composition épaississante présente une thixotropie.

10. Composition épaississante selon l'une quelconque des revendications 1 à 6 et 9, dans laquelle la composition épaississante contient 1 % en masse de fibres de cellulose et 0,01 % en masse de l'électrolyte, **caractérisée en ce que** dans des mesures de viscoélasticité dynamique utilisant une composition épaississante contenant 1 % en masse de fibres de cellulose et 0,01 % en masse d'un électrolyte, un coefficient de viscosité dynamique η' mesuré dans un cône/plan selon un déplacement dynamique de 0,5 degré et une fréquence de 1 Hz dans un état statique à 25 °C pendant 2 heures est d'au moins 150 % de η' à 25 °C pendant 0 heure.

11. Cosmétique comprenant la composition épaississante selon l'une quelconque des revendications 1 à 6 et 9 à 10.

12. Cosmétique selon la revendication 11, comprenant en outre un composant aqueux.

13. Cosmétique selon la revendication 11 ou 12, comprenant en outre une proportion de 1 partie en masse à 100 parties en masse d'un ou de deux polyalcools ou plus choisis dans le groupe constitué du 1,3-butylèneglycol, du glycérol et du diglycérol par rapport à 1 partie en masse des fibres de cellulose.

14. Cosmétique selon l'une quelconque des revendications 11 à 13, comprenant en outre un composant huileux.

15. Cosmétique selon l'une quelconque des revendications 11 à 14, comprenant en outre un tensioactif.

16. Cosmétique selon la revendication 14 ou 15, dans lequel le cosmétique se présente sous la forme d'une émulsion.

17. Préparation externe comprenant la composition épaississante selon l'une quelconque des revendications 1 à 6 et 9 à 10.
